# EUROPÄISCHE PATENTSCHRIFT

(11) **EP 3 727 183 B1**
(45) Veröffentlichungstag und Bekanntmachung des Hinweises auf die Patenterteilung: **30.11.2022**
(21) Anmeldenummer: 18833211.8
(22) Anmeldetag: 19.12.2018
(51) Int. Cl.: A61C 5/50

(54) **WURZELKANALFÜLLUNGSZUSAMMENSETZUNG**
ROOT CANAL FILLING COMPOSITION
COMPOSITION DE REMPLISSAGE DU CANAL RADICULAIRE

(30) Priorität: 22.12.2017 EP 17210071; 22.12.2017 DE 102017131135; 22.12.2017 DE 202017107865 U
(43) Veröffentlichungstag der Anmeldung: 28.10.2020
(73) Patentinhaber: Lietzau, Markus, 14109 Berlin (DE)
(72) Erfinder: Lietzau, Markus, 14109 Berlin (DE)
(74) Vertreter: ETL IP Patent- und Rechtsanwaltsgesellschaft mbH
(86) Internationale Anmeldenummer: PCT/EP2018/085981
(87) Internationale Veröffentlichungsnummer: WO 2019/122009

(56) Entgegenhaltungen:
- EP-A1- 0 644 743
- WO-A1-00/67659
- WO-A1-2013/128952
- WO-A1-2014/115090
- US-A1- 2005 066 854

## Beschreibung

Die Erfindung betrifft eine Wurzelkanalfüllzusammensetzung zur Anordnung in einem Zahnwurzelkanal, ein Set zur Bereitstellung der benötigten Materialien sowie ein Verfahren zur Applikation der Materialien.

Material der Wahl zum bakteriendichten Verschluss des Wurzelkanalsystems bei Zähnen ist derzeit Guttapercha mit Sealern auf Zinkoxid-Eugenol-, Kalziumhydroxid-, Silikon-, oder Epoxidharz-Basis. Das System Guttapercha/Sealer erfüllt weitgehend alle Anforderungen, die an ein Wurzelkanalfüllungsmaterial zu stellen sind, wie Biokompatibilität, chemische Stabilität, Homogenität, Radioopazität, geringe Techniksensibilität sowie die Möglichkeit der Revision. Jedoch lässt sich mit Guttapercha kein vollständig bakteriendichter Verschluss des Wurzelkanalsystems erzielen. Eine Bakterienpenetration kann entlang der Grenzflächen Dentin -Sealer oder Sealer-Guttapercha stattfinden, da Guttapercha weder mit dem Wurzelkanaldentin noch mit dem Sealer einen Verbund eingeht.

In der Endodontie gewinnen daher adhäsive Sealer gegenüber konventionellen Sealern an Popularität. Eine Hybridisierung zwischen Kollagen (Wurzelkanaldentin) und Kunststoff (Sealer auf Methacrylatbasis) soll zum einen den bakteriendichten Verschluss des Wurzelkanalsystems ermöglichen, zum anderen wird - wie in koronalen Kavitäten - eine Erhöhung der mechanischen Widerstandsfähigkeit durch den adhäsiven Verbund diskutiert. Anders als am koronalen Dentin können jedoch Parameter wie anatomische Besonderheiten des Wurzelkanaldentins; die Polymerisationsschrumpfung des Sealers, sowie der hohe C-Faktor (Relation gebundener zu freier Oberfläche) im Wurzelkanal die Integrität des adhäsiven Verbundes zum Wurzelkanaldentin beeinträchtigen und einer Infektion Vorschub leisten.

Ein weiteres Problem der adhäsiven Wurzelkanalfüllung aus kunststoffbasiertem Sealer/Guttapercha und damit eine mögliche Eintrittspforte für Bakterien stellt die fehlende chemische Verbindung des kunststoffbasierten Sealers mit der Polyisopren-Komponente der Guttapercha dar.

Derzeit gibt es zwei Strategien für die adhäsive Wurzelkanalfüllung. Eine Strategie, methacrylat-basierte Sealer an Guttapercha zu binden, bedient sich der Beschichtung konventioneller Guttapercha-Spitzen mit Kunststoffkomponenten (Polybutadien-Diisocyanat-Methacrylat). Bei der Beschichtung (10 bis 15µm) wird die Verbindung des Polyisoprens der Guttapercha zum Methacrylat im Sealer über ein bifunktionelles Diisocyanat vermittelt, das über eine hydrophobe Gruppe einerseits an das Polyisopren der Guttapercha bindet und über eine hydrophile Methacrylatgruppe andererseits den Verbund zum Sealer vermittelt. Auch ohne die Verwendung eines Primers kommt es mit dem hydrophilen Sealer zu einer ausgeprägten Ausbildung von Tags in den Dentinkanälchen und einer Hybridschicht.

Ein zweiter Ansatz befasst sich damit, ein gefülltes, thermoplastisches Komposit als Obturationsmaterial zu schaffen, das in seinen Eigenschaften Guttapercha ähnelt. Durch die inkorporierten Methacrylate wird die Anbindung an den methacrylatbasierten Sealer gewährleistet. Resilon ist ein synthetisches Wurzelkanalfüllungsmaterial, bestehend aus Polycaprolacton (Polyester-Polymer), Dimethacrylaten, sowie Glas- und radioopaken Füllern. Resilon ist weich und thermoplastisch wie Guttapercha. Das kunststoff-basierte Material ist entweder in Form von Stiften oder Pellets erhältlich und kann mit den bekannten Obturationstechniken verwendet werden. Nach der Aufbereitung des Wurzelkanalsystems wird Resilon mittels eines selbstätzenden Primers und eines dualhärtenden, methacrylat-basierten, hydrophilen Sealers adhäsiv an das Wurzelkanaldentin gebunden, um koronal den Aufbau zu stabilisieren. Allerdings hat sich in Langzeitstudien gezeigt, dass Resilon aufgrund von Änderungen in den physikalischen Eigenschaften zunehmend undicht wird, so dass sich Hohlräume und Kanäle in der Füllung oder zwischen Füllung und Zahnsubstanz ausbilden, die die Ansiedlung von Keimen begünstigen.

Allerdings kann es beim Kontakt endodontischer Sealer mit den periapikalen Hart- und Weichgeweben zu lokalen oder auch systemischen Reaktionen kommen (Geurtsen 2001). Bei kunststoff-basierten Sealern können die Inhaltstoffe zytotoxisch oder mutagen wirken. EP 0 644 743 B1 offenbart eine Wurzelkanalzusammensetzung zur Anordnung in einem Zahnwurzelkanal nach dem Oberbegriff von Anspruch 1.

Der Erfindung liegt nun die Aufgabe zugrunde, die Probleme des Stands der Technik zu überwinden und insbesondere eine bakteriendichte Wurzelkanalfüllung bereitzustellen, die die bekannten Nebenwirkungen der Komposite bzw. Sealer nicht hat.

Die Aufgabe wird durch eine Wurzelkanalfüllungszusammensetzung sowie ein Verfahren und ein Set zu deren Herstellung mit den Merkmalen der unabhängigen Ansprüche gelöst.

Somit betrifft ein erster Aspekt der Erfindung eine Wurzelkanalfüllungszusammensetzung zur Anordnung in einem Zahnwurzelkanal. Die erfindungsgemäße Wurzelkanalfüllungszusammensetzung, im Weiteren auch Zusammensetzung oder Wurzelfüllung genannt, umfasst einen Glasfaserstift mit einem ersten und einem zweiten Ende, wobei an dem ersten Ende ein apikaler Stopp aus einem physiologischem Material angeordnet ist und der Glasfaserstift zumindest bereichsweise vollflächig von einem Füllmaterial umschlossen ist. Erfindungsgemäß umfasst das Füllmaterial ein dentin-adhäsives Füllmaterial oder besteht aus einem solchen.

Der Vorteil der erfindungsgemäßen Zusammensetzung besteht darin, dass eine bakteriendichte Wurzelkanalfüllung angeboten werden kann, die zudem absolut verträglich für das umgebende Gewebe ist. Dies wird durch die Kombination aus physiologischem apikalem Stopp und dentin-adhäsivem Verschluss erzielt.

Unter physiologisch ist vorliegend ein Material zu verstehen, welches keine oder nur kurzfristig sehr geringe schädliche Einflüsse auf lebendes Gewebe zeigt, wobei vorliegend insbesondere auf das zum Apex benachbarte Gewebe, also im Wesentlichen den Zahnhalteapparat abgestellt wird. Der apikale Stopp bildet eine Barriere zwischen lebendem Gewebe und Füllmaterial und verhindert somit einen Kontakt und eine chemische oder physiologische Wechselwirkung zwischen Füllmaterial und Gewebe. Darüber hinaus verhindert der Stopp, dass Füllmaterial aus dem Apex in das umgebende Gewebe austritt. Die Anordnung des apikalen Stopps verhindert somit bei der Kombination mit dentin-adhäsivem Füllmaterial kurzfristige und langfristige Schädigungen im Zahnhalteapparat. Daher ist die erfindungsgemäße Zusammensetzung auch zur Wurzelkanalfüllung bei entzündeten Apizes oder Knochenläsionen im apikalen Bereich verwendbar.

Unter "bereichsweise vollflächig umschlossen" ist vorliegend ein Bereich des Glasfaserstifts ausgehend vom ersten Ende zu verstehen, der über seinen gesamten Umfang das Füllmaterial aufweist. Die Einschränkung bereichsweise bezieht sich auf die Länge des Glasfaserstifts und verdeutlicht, dass insbesondere in einem zum zweiten Ende benachbarten Bereich kein Füllmaterial angeordnet ist, insbesondere um eine Handhabung des Glasfaserstifts zu ermöglichen. Insbesondere ist das Füllmaterial ausgehend vom apikalen Stopp vom ersten Ende aus vollflächig über eine Länge des Glasfaserstifts im Bereich von 85% bis 99,9% der Kanallänge, insbesondere im Bereich von 90% bis 99% der Kanallänge, beziehungsweise über eine Länge im Bereich von 5 mm bis 23 mm, insbesondere im Bereich von 8 mm bis 19 mm angeordnet.

Das dentin-adhäsive Füllmaterial ist ein Material, welches aufgrund adhäsiver Wechselwirkungen im nicht ausgehärteten Zustand in die Detintubuli des Zahns eindringt und diese verschließt. Dadurch kommt es auf zweierlei Art und Weise zu einem bakteriendichten Verschluss. Zum einen können keine Keime über die Dentintubuli von außen in die Zahnwurzel gelangen. Zum anderen wird eine Spaltbildung zwischen Dentin und Füllmaterial unterbunden, wodurch auch keine Keime aus der Mundhöhle oder aus dem apikalen Bereich in einen derartigen Spalt eindringen können. Da Bakterien zur Vermehrung Raum und Nahrung benötigen ("bacteria needs food and space") ist den Keimen jegliche Vermehrungsgrundlage genommen. Die die erfindungsgemäße Zusammensetzung bietet daher eine deutliche Reduzierung mittel- und langfristiger Komplikationen, da zum einen durch das dentin-adhäsive Material auslösbare Entzündungsprozesse unterbunden werden (kurz-bis mittelfristige Komplikationen) und zum anderen eine Neuansiedlung von Bakterien in verbliebenen Freiräumen (mittelfristige bis langfristige Komplikationen) verhindert werden können.

Eine dentin-adhäsive Befestigung ist ein Behandlungsverfahren in der Zahnmedizin zur Befestigung von Füllungsmaterial oder Zahnersatz am Zahn. Dabei wird die Dentinoberfläche einer Kavität chemisch vorbehandelt, indem dünnflüssige Dentinhaftvermittler in die Oberflächenstrukturen eindringen können und nach chemischer Aushärtung einen mikromechanischen Verbund zwischen Dentin und einer Kompositfüllung eingehen. Dadurch kann auf makromechanische Retentionen verzichtet werden, die einen größeren Zahnsubstanzverlust nach sich ziehen.

Darüber hinaus führt die Vermeidung einer Spaltbildung zwischen Dentin und Füllmaterial zu einer Stabilisierung des Zahnes und verhindert eine erhöhte Brüchigkeit.

Komposite (lateinisch compositum ,das Zusammengesetzte') sind zahnfarbene plastische Füllungsmaterialien für die zahnärztliche Behandlung. Umgangssprachlich werden die seit den 1960er Jahren verwendeten Werkstoffe auch oft als Kunststofffüllungen bezeichnet. Das zahnmedizinische Einsatzgebiet der Komposite sind Füllungen oberhalb des Wurzelkanals und die Befestigung von Keramikrestaurationen, Kronen und Wurzelstiften.

Komposite bestehen aus einer organischen Kunststoffmatrix, die mit anorganischen Füllkörpern versetzt ist. Die Anwendung der Komposite erfolgte anfangs fast ausschließlich im Frontzahnbereich. Inzwischen werden Komposite mit einem erhöhten Füllkörpergehalt auch im Seitenzahnbereich eingesetzt. Die Weiterentwicklung der Haftvermittler (Bonding) und die Dentin-Adhäsivtechnik ermöglichte den Einsatz im Seitenzahnbereich.

Abwandlungen der Komposite, die vorliegend auch unter dem Begriff Komposit verstanden werden sollen, sind Compomere, Ormocere und Glasionomerzemente. Wobei letztere formal einer anderen Werkstoffklasse angehören und teilweise die obengenannten Materialien umfassen.

Die Verarbeitung von Füllungsmaterial auf Kompositbasis, also eines Materials, welches bezogen auf das Volumen zu einem überwiegenden Teil aus Komposit besteht, ist im Vergleich zu einer Amalgamfüllung aufwendiger und zeitintensiver, da es in mehreren Schichten aufgetragen und schichtweise mit einer Polymerisationslampe gehärtet wird, um die Polymerisationsschrumpfung des Materials zu verringern. Voraussetzung für eine dauerhaft dichte Kompositfüllung ist die adhäsive Befestigung am Zahn. Diese wird beispielsweise durch ein Anätzen, insbesondere mit Phosphorsäure und ein anschließendes Auftragen eines Adhäsivs erzielt.

Die Matrix von Kompositen besteht insbesondere aus Kunststoffen auf Methacrylatbasis. Daneben können auch Spuren von Formaldehyd, Glutaraldehyd und Säuren enthalten sein.

Bevorzugt weist das Füllmaterial Füllstoffe auf, welche eine Schrumpfung des Materials reduzieren oder sogar verhindern können. Als Füllstoffe kommen Glas-, Keramik und Quarzteilchen (Silikate, Sande) zum Einsatz. Diese weisen weiter bevorzugt eine Beschichtung mit Silanen auf, die deren Verbindung mit dem Kunststoff verbessert. Die Silanisierung dient mit anderen Worten als Verbundphase zwischen der organischen und der anorganischen Matrix. Silane können Glas an eine organische Matrix chemisch binden. Einerseits gehen die Silanolgruppen des Silans eine Kondensationsreaktion mit der Glasoberfläche der Füllstoffe ein. Andererseits erfolgt eine kovalente Bindung der Methacrylsäuregruppe des Silans mit dem Matrixkunststoff der organischen Phase.

Die Füllstoffe werden als anorganische Phase der Komposite bezeichnet. Vorliegend sind Füllstoffe ausgewählt aus: Gläser bzw. Glaskeramiken (z. B. Barium-Aluminium-Glas), Silikate, Siliziumdioxid.

Die organische Phase der Komposite ist mit Vorteil ein Methacrylat (Acryl), welches insbesondere lichtgehärtet wird. Es werden Hydroxyethylmethacrylat (HEMA), Triethylenglycoldimethacrylat (TEGDMA) oder Bisphenolglycidylmethacrylat (BisGMA) verwendet.

Neben dem eigentlichen Monomer als Hauptbestandteil der organischen Phase sind Komposite, wie BisGMA-Komposite bevorzugt, die noch weitere insbesondere funktionale, Bestandteile enthalten. Dabei sind die weiteren Bestandteile auszuwählen aus der Gruppe: Mono-, Di- und Triacrylate (als Comonomer) - siehe: Copolymer, Campherchinon oder Phenylpropandiol (als Initiator der Photopolymerisation nach Beleuchtung mit dem blauen Licht einer Polymerisationslampe), Toluidin (als Akzelerator der Photopolymerisation), Hydrochinon (als Inhibitor der Photopolymerisation, damit diese nicht bereits durch normales Tageslicht startet), Benzophenon (als UV-Stabilisator, damit die Kunststofffüllung im Laufe der Jahre im Patientenmund farbstabil bleibt), Farben und Pigmente (zur Farbgebung für die Kunststofffüllung).

In einer bevorzugten Ausführung umfasst das Füllmaterial Komposite auszuwählen aus der Gruppe: hochvisköse, stopfbare Komposite - hoher Fülleranteil, niedrigvisköse, fließfähige Komposite - reduzierter Füllkörperanteil - als Zwischenschicht unter den stopfbaren Kompositen. Weiter bevorzugt erfolgt die Polymerisation des Komposits chemisch und/oder durch Licht.

Dual härtende Komposite (also sowohl licht- als auch chemisch polymerisierbare Komposite) werden eingesetzt, wenn die Zuführung des Lichts zum Kompositmaterial teilweise ausgeschlossen ist und sind daher für die Verwendung in der erfindungsgemäßen Zusammensetzung besonders bevorzugt. Diese werden dann beispielsweise nur an den erreichbaren Rändern (zusätzlich) mit Licht ausgehärtet, da die tiefer liegenden Schichten einer Beleuchtung nicht zugänglich sind, während an den für das Licht unzugänglichen Stellen eine chemische Polymerisation stattfindet.

Mit Vorteil umfasst das Füllmaterial ein Komposit, insbesondere ein chemisch, ein dual- oder ein lichthärtendes Komposit.

Weiter vorteilhaft ist das Komposit ein selbstadhäsives Komposit. Dies reduziert zum einen die Zahl der Komponenten der Zusammensetzung und zum Zweiten die die Behandlungszeit beim Einbringen der Zusammensetzung in einen Zahn, da zumindest auf ein Auftragen von eines sogenannten Primers und gegebenenfalls auf ein vorbereitendes Ätzen verzichtet werden kann.

In einer bevorzugten Ausgestaltung der Erfindung ist vorgesehen, dass der Glasfaserstift ein-oder mehrteilig ausgebildet ist. Unter mehrteiliger Ausgestaltung ist vorliegend zu verstehen, dass mehrere nicht stoffschlüssig miteinander verbundene, nebeneinander angeordnete längserstreckte Teile den Glasfaserstift ausbilden. Die Einzelteile sind bevorzugt mit einer Halterung, beispielsweise einem den Umfang des Glasfaserstifts umlaufenden Ring oder einer Kappe, lösbar verbunden. Selbstverständlich umfasst auch ein einteiliger Glasfaserstift mehrere Fasern, die dann allerdings stoffschlüssig miteinander verbunden sind. Der Vorteil eines einteiligen Stifts liegt insbesondere in einer unkomplizierteren Handhabung. Dahingegen zeichnet sich ein mehrteiliger Glasfaserstift durch eine erhöhte Biegsamkeit, eine höhere Dickenvariabilität, durch Entfernen oder Kombinieren einzelner Teile, sowie eine bessere Anpassbarkeit an sich verjüngende Wurzelkanäle aus. So werden beispielsweise in die Spitze eines sich verjüngenden Wurzelkanals nur einige der Teile hineingeschoben, während die anderen erst in einer Höhe mit breiterem Wurzelkanal beginnen. Dies ist für einfache einteilige Stifte nicht möglich. Dieser Nachteil wird vorteilhafterweise durch eine verjüngende Spitze des ersten Endes eines einteiligen Glasfaserstifts ausgeglichen, obschon eine flexible Anpassung an diverse Wurzelkanalgeometrien wie sie bei mehrteiligen Stiften gegeben ist nicht erreicht werden kann.

Dual härtende Komposite haben nach dem Aushärten immer noch einen sehr hohen Restmonomergehalt von bis zu 45 %. Dieser steht im Verdacht Nebenwirkungen für den Patienten zu entwickeln. Um diesen entgegenzuwirken ist in einer weiteren Ausgestaltung der Erfindung der Glasfaserstift zumindest bereichsweise, insbesondere in einem dem apikalen Stopp benachbarten Bereich, derart beschaffen, dass er einen seitlichen Lichtaustritt ermöglicht. Bei einer Beleuchtung der Zusammensetzung nach der Anbringung im Zahn wird ein Teil des Lichts am zweiten Ende in den Glasfaserstift eingebracht und durch diesen hindurch an das erste Ende geleitet. Auch an einem herkömmlichen Glasfaserstift tritt das Licht an diesem Ende wieder aus. Eine Beschaffenheit des Glasfaserstifts derart, dass zusätzlich ein seitlicher Lichtaustritt ermöglicht ist führt dazu, dass ein größerer Bereich, insbesondere ein Bereich im Innern des Wurzelkanals, für Licht zugänglich wird. Der Polymerisationsgrad wird deutlich erhöht und ein Monomerengehalt reduziert. Zudem können auch Komposite verwendet werden, die überwiegend lichtgehärtet werden.

Dessen ungeachtet ermöglicht die erfindungsgemäße Ausgestaltung sicherzustellen, dass das Kompositmaterial weder apikal noch lateral Kontakt mit lebendem Gewebe des Patienten hat.

Vorzugsweise wird eine derartige Beschaffenheit des Glasfaserstifts erreicht, indem der Glasfaserstift im betreffenden Bereich angeraut ist und/oder regelmäßige oder unregelmäßige Erhebungen und Vertiefungen aufweist, beispielsweise in Form eines Waben- oder Kastenmusters. Derartige Ausgestaltungen können zu einer Stabilitätsreduzierung im Glasfaserstift führen, die jedoch durch die Bindung des Komposits ausgeglichen werden kann. Zusätzlich kann die Stabilität erhöht werden, indem nur einige der Teile eines mehrteiligen Glasfaserstifts das Merkmal aufweisen.

Alternativ oder zusätzlich werden die Fasern eines mehrteiligen Glasfaserstifts in unterschiedlichen Längen ausgeführt, wobei die einzelnen Teile am zweiten Ende bündig abschließen. Am ersten Ende wird dann auf unterschiedlicher Höhe ein Lichtaustritt am jeweiligen Ende der Glasfaser erreicht. Dies führt zu einem breiteren Bereich für Lichtaustritt und zu einer größeren lichtgehärteten Oberfläche. Diese Ausgestaltung ermöglicht eine vergrößerte Lichtaustrittsfläche ohne die Stabilität des Stifts zu reduzieren.

In weiter bevorzugter Ausgestaltung der Erfindung ist vorgesehen, dass der apikale Stopp ein biologisch resorbierbares Material umfasst oder aus einem solchen besteht. Dies hat den Vorteil, dass eine maximale physiologische Verträglichkeit gegeben ist.

Mit besonderem Vorteil ist dies gegeben, wenn der apikale Stopp einen biokompatiblen Gewebe- oder Knochenkleber aufweist. Dies führt dazu, dass nicht nur eine Reizung des umliegenden Gewebes vermieden wird, sondern durch die erfindungsgemäße Zusammensetzung bei der Applikation in einem Wurzelkanal sogar Knochenläsionen im apikalen Bereich des Zahnhalteapparats geheilt werden, oder zumindest deren Heilung unterstützt wird.

Besonders bevorzugt umfasst der apikale Stopp Alginate, Collagene, eine Hyaluronsäurezusammensetzung, sowie medizinische Gewebekleber wie Cyanoacrylate, Calciumphosphate, insbesondere Hydroxylapatit, Poly-Lactone, insbesondere Poly-Caprolactone wie Poly(glykolsäure-co-caprolacton oder Poly(L-Laktid-co-caprolacton Poly-Laktide wie Poly(L-Laktid-co-DL-Laktid) und Poly(DL-Laktid-co-glykolid), Acylacrylnitril, Dialkylmethylenmanolat, α-substituierte Vinylidenealkylsulfonate, Sulfonate, Oligo-p-dioxanon und/oder deren Derivate.

In der Gruppe der Cyanoacrylate sind besonders alkyl-Cyanoacrylate, vorzugsweise mit einer alkyl-Kettenlänge im Bereich von 1 bis 12, besonders bevorzugt mit einer Kettenlänge im Bereich von 2 bis 8 zur Verwendung als apikaler Stopp bevorzugt. Diese haben den Vorteil, dass sie mit milden Basen, wie Wasser, welches bei erfindungsgemäßer Anwendung stets vorhanden ist, ohne weitere Polymerisationsauslöser vollständig aushärten. Die zusätzliche Anwendung von Wärme und/oder Licht zum Aushärten ist nicht erforderlich. Die Eigenschaften des apikalen Stopps sind bei Verwendung von Cyanoacrylaten durch deren Alkylkettenlänge einstellbar. Mit steigender Kettenlänge nimmt die Verträglichkeit sowie die Härte des Klebers ebenso wie dessen Aushärtezeit zu.

Cyanoacrylate sind zur erfindungsgemäßen Verwendung als apikaler Stopp mit Vorteil mit elektronenreichen Olefinen, wie 4-methoxystyren vermischt um beim Aushärten mit diesen zu (co-)polymerisieren.

Mit Vorteil umfasst der apikale Stopp neben den genannten härtbaren Materialien funktionale Zusatzstoffe. Hierzu zählen vorzugsweise Stabilisatoren, Verdickungsagentien, Bindemittel, Polymerisationsinhibitoren wie Schwefeldioxid, Stickoxid, O-Schwefelbezoesäureanhydrid und/oder Phosphorpentoxid und/oder Weichmacher.

Alternativ oder zusätzlich weist der apikale Stopp zusätzlich anorganische Nanopartikel, beispielsweise aus SiO₂ oder APOSS (acrylate polyhedral oligomeric silsesquioxane) bestehende oder diese umfassend, auf. Deren Zugabe ist geeignet die Zellverträglichkeit des apikalen Stopps weiter zu verbessern.

Weiter bevorzugt ist, wenn der apikale Stopp vorgeformt oder als Vorläufer in pastöser, also nur teilgehärteten Form vorliegt. Die Aushärtung erfolgt dann insbesondere durch Lichthärtung mittels Einbringung von Licht über den Glasfaserstift und/oder durch Aushärtung eines 2K-Gemisches. Liegt der Stopp vorgeformt vor, wird darunter ein flexibler, insbesondere komprimierbarer Pfropfen verstanden, der sich beim Einbringen in den Wurzelkanal derart komprimieren lässt, dass er bis in den apikalen Bereich vordringt und gegebenenfalls am Bestimmungsort wieder teilweise expandiert und dabei den Apex verschließt.

Die Einbringung des apikalen Stopps erfolgt dann bis zur Konstriktion unter deren kompletten physiologischen Erhalts.

In weiterer bevorzugter Ausgestaltung der Erfindung ist vorgesehen, dass der apikale Stopp eine Länge von nicht mehr als 1/10, insbesondere nicht mehr als 1/20, vorzugsweise nicht mehr als 1/30 der Länge des Glasfaserstifts aufweist. Dies führt dazu, dass der apikale Stopp nach der Einbringung in den Wurzelkanal weniger als 1/10, insbesondere weniger als 1/15, vorzugsweise nicht mehr als 1/20 in den Wurzelkanal hineinreicht. Das entspricht im Wesentlichen der Arbeitslänge, also +/- 0, wobei bevorzugt sogar -0,5 bis -1mm beim Überstopfen erzielt werden. Was den Vorteil bringt, dass ein größtmöglicher Teil des Wurzelkanals mit dem dentin-adhäsiven Füllmaterial gefüllt wird, und somit möglichst alle Dentintubuli verschlossen sind und der Zahn über die gesamte Länge stabilisiert ist und keine Hohlräume oder instabile Bereiche aufweist in denen komprimierbares oder resorbierbares Material angeordnet ist, was mittelbar zu Hohlräumen und somit Instabilitäten und/oder Bakterienbesiedelung führen würde.

Ein weiterer Aspekt der Erfindung betrifft ein Set zur Herstellung der erfindungsgemäßen Wurzelkanalfüllzusammensetzung. Das erfindungsgemäße Set umfasst einen Glasfaserstift insbesondere in einer der oben beschriebenen Ausgestaltungen als prothetische Hochstabilisierung und Anker für die Deckfüllung, einen apikalen Stopp, vorzugsweise in einer oben beschriebenen Ausgestaltungen oder eines Vorläufers davon, sowie ein dentin-adhäsives Füllmaterial in einer der oben beschriebenen Ausgestaltungen oder dessen Vorläufer.

Das erfindungsgemäße Set macht mit Vorteil die oben beschriebene Wurzelkanalzusammensetzung mit all den dort beschriebenen Vorteilen herstellbar. Die Darbietung als Set hat darüber hinaus den Vorteil, dass alle notwendigen Edukte und Werkzeuge umfasst sind und somit bei zur Durchführung einer Zahnwurzelkanalfüllung lediglich das erfindungsgemäße Set bereitgestellt werden muss und nicht in zeitaufwendiger Suche Einzelsubstanzen zusammengesucht werden müssen. Ferner werden stets alle Ausgangsstoffe gleichzeitig verbraucht und bestellt. All dies führt zu einem besonders effektiven Zeitmanagement beim Behandler. Darüber hinaus finden sich Planungs- und Synergievorteile auf Produzentenseite, was wiederum eine Kostensenkung möglich macht, die sich produzenten- und behandlerseitig bemerkbar macht.

Vorliegend soll unter Vorläufer eine Verbindung oder ein Material verstanden werden, dass sich chemisch oder physikalisch insbesondere beim Applizieren in den erfindungsgemäßen Stopp beziehungsweise das Füllmaterial überführen lässt. Insbesondere sind darunter 2-Komponenten-Werkstoffe zu verstehen oder solche die an Luft insbesondere chemische Reaktionen eingehen.

Das Füllmaterial liegt mit besonderem Vorteil vorgemischt und nicht oder nur teilweise ausgehärtet vor. Dazu ist es im vorbestimmten Zustand applikationsfertig vor. Zum Schutz vor chemischer Abreaktion und vor Bakterien etc. ist das Füllmaterial luft- und. Insbesondere licht-dicht verschlossen. Hierbei die Darbietung in einer Kanüle oder Spritze bevorzugt, die insbesondere einen Applikator aufweist.

Der apikale Stopp ist je nach gewähltem Material bevorzugt als komprimierbarer, elastischer Pfropf oder Faden vorgefertigt im Set vorhanden. Hierbei weist der Propf Ausmaße beziehungsweise der Faden Durchmesser im Bereich von 0,1 bis 8 mm, insbesondere im Bereich von 0,2 bis 1,5 mm, vorzugsweise im Bereich von 0,3 bis 1 mm auf. Die Größe des Pfropfes variiert vorzugsweise in Abhängigkeit vom verwendeten Material, insbesondere von dessen Komprimierbarkeit. Besonders bevorzugt ist die Verwendung eines Kollagenpfropf mit einem Anteil Hydroxylapatit in annähernd kubischer Form mit einem Kantenmaß von im Bereich von 0,3 bis 1mm.

Alternativ oder zusätzlich ist zur Verwendung als apikaler Stopp je nach Materialzusammensetzung eine insbesondere pastös, also viskos vorliegende Zusammensetzung im Set enthalten, die dann über ein geeignetes Werkzeug, insbesondere eine Kanüle beziehungsweise eine Spritze an den apikalen Bestimmungsort gebracht wird. Dies ist vorzugsweise bei Verwendung der genannten medizinischen Gewebekleber, wie alkyl-Cyanoakrylat-Zusammensetzungen oder deren Vorläufer der Fall.

In weiter bevorzugter Ausgestaltung weist der apikale Stopp auf zumindest einer Seite, eine unregelmäßige, insbesondere aufgeraute und/oder geriffelte Oberfläche auf. Diese Seite entspricht vorzugsweise einer Oberfläche, die bei bestimmungsgemäßem Gebrauch nach apikal gerichtet, also einer vom Wurzelkanal abgewandten und einem Zahnhalteapparat, insbesondere einem Knochen zugewandten Seite des Stopps. Diese Ausführung hat den Vorteil, dass sich bei Verwendung der erfindungsgemäßen Zusammensetzung Makrophagen besser an der Oberfläche des Stopps anlagern und somit eine Heilung von Knochenläsionen beschleunigt werden kann.

Alternativ liegt der apikale Stopp in Form einer Paste und/oder in Pulverform vor. Insbesondere in letzter Variante erfolgt vor dem Applizieren eine Vormischung.

Es soll verstanden werden, dass das erfindungsgemäße Set in der Endodontologie eingesetzt wird und insbesondere nicht in der Prothetik. Das erfindungsgemäße Set unterscheidet sich von einem in der Prothetik verwendeten insbesondere in dem Bestandteil des apikalen Stopps.

Im Unterschied zu prothetischen Stiften werden vorliegend deutlich dünnere Glasfaserstifte verwendet mit einem Durchmesser im Bereich eines Wurzelkanals, insbesondere im Bereich von 0,2 bis 0,75 mm, bevorzugt 0,3, 0,45 oder 0,6mm. Mit anderen Worten, der verwendete Glasfaserstift überschreitet einen Durchmesser von 0,65 mm beziehungsweise von 0,3 mm nicht. Vorteilhafterweise kann zusätzlich zur Wurzelkanalfüllung eine Verankerung erzielt werden, wenn der Glasfaserstift länger als der Wurzelkanal ausgeführt ist.

Alternativ liegt das Füllmaterial und/oder der apikale Stopp in einzeln verpackten nicht reaktiven Einzeledukten vor, die vor Applikation zunächst angemischt werden. Dabei ist bevorzugt, wenn alle Reaktionsteilnehmer in äquivalenten Mengen im Set enthalten sind.

In einer vorteilhaften Ausführung des erfindungsgemäßen Sets ist vorgesehen, dass dieses einen dentin-adhäsiven Primer und/oder ein Ätzmittel umfasst. Der Primer ist insbesondere dann vorgesehen, wenn es sich bei dem Komposit nicht um ein selbst-adhäsives Komposit handelt.

Das Ätzmittel ist vorzugsweise pastös oder flüssig und ebenfalls in einer Tube oder Spritze mit Applikator abgefüllt.

In einer weiter bevorzugten Ausführung der Erfindung liegen der Glasfaserstift, der apikale Stopp oder dessen Vorläufer und das dentin-adhäsive Füllmaterial oder dessen Vorläufer räumlich getrennt voneinander, insbesondere einzeln verpackt, in dem Set angeordnet vor.

Alternativ liegen die genannten Bestandteile in Form der erfindungsgemäßen Wurzelkanalfüllzusammensetzung vor, wobei das an dem Glasfaserstift angeordnete Füllmaterial zusätzlich derart isoliert umschlossen ist, dass es am Glasfaserstift insbesondere mit Kontakt zu diesem fixiert ist, und insbesondere hermetisch und steril abgeschlossen ist. Dies ist beispielsweise durch eine angeschweißte Umhüllung, bspw. In Form eines Kunststoffs, möglich. Die Umhüllung wird dabei derart angeordnet, dass der apikale Stopp nicht mit umhüllt ist, sondern vielmehr von dem Füllmaterial separiert ist, um sicherzustellen, dass der apikale Stopp nicht mit dem unter Umständen nicht physiologischem Füllmaterial kontaminiert wird. Diese Ausgestaltung bietet den Vorteil, dass die Zusammensetzung mit wenigen Handgriffen unter enormer Zeitersparnis vorliegt und direkt appliziert werden kann.

Beide Ausgestaltungen bieten den Vorteil, dass keine Abmessung der Edukte erfolgen muss oder Restbestände entstehen, die wiederum gelagert werden müssen. Dabei ist unabhängig von den Ausgestaltungen des Sets zu verstehen, dass die einzelnen Verpackungen innerhalb des Sets stets hermetisch, insbesondere aber steril sind.

In der alternativen Ausführung ist es zudem von Vorteil, wenn zudem noch Füllmaterial und/oder Material des apikalen Stopps separat verpackt im Set vorliegt um nach einem Applizieren der Wurzelkanalfüllung im Wurzelkanal eventuelle Freiräume aufzufüllen.

Mit Vorteil ist am zweiten Ende des Glasfaserstifts eine Halterung in Form einer Kappe oder eines leicht lösbaren Rings angeordnet.

In weiter bevorzugter Ausgestaltung ist vorgesehen, dass das Set jedes der Bestandteile in einer Menge umfasst, die die Füllung genau eines Zahns ermöglicht. Dabei kann das Set grundsätzlich in verschiedenen Größen vorliegen, wobei eine Größe jeweils an den Bedarf eines Zahntyps angepasst ist. Das heißt insbesondere, dass die Setgröße mit Vorteil auf die Anzahl der Zahnwurzeln bzw. -kanäle abgestimmt ist. In den unterschiedlichen Setausführungen variieren dann insbesondere die Menge an Komposit, apikaler Stopps und die Größe (Dicke) und Menge an Glasfaserstiften.

Alternativ umfasst das Set die einzelnen Bestandteile in einer Menge für mehrere Anwendungen. Dies hat unter anderem den Vorteil, für unterschiedliche Zähne (Front- und Backenzähne) das selbe Set verwendet werden kann. Es ist nicht erforderlich mehrere unterschiedliche Sets zu bevorraten. Darüber hinaus ist ein größerer Maßstab aus ökologischen und ökonomischen Gesichtspunkten für den Verbraucher attraktiver.

Ein weiterer Aspekt der vorliegenden Erfindung ist ein Verfahren zum Anbringen einer erfindungsgemäßen Wurzelkanalfüllungszusammensetzung ex-vivo, insbesondere unter Verwendung des erfindungsgemäßen Sets.

Vorliegend wird das Anbringen der Wurzelkanalfüllzusammensetzung, insbesondere ex-vivo, also beispielsweise bei einem extrahierten Zahn, durchgeführt.

In jedem Fall ist eine vorheriger Eröffnung des Zahns über den apikalen Bereich hinaus erfolgt. Das erfindungsgemäße Verfahren umfasst die folgenden Schritte in der angegebenen Reihenfolge:
a) Zunächst wird der apikale Stopp aus dem physiologischen Material in den Kanal bis zum Apex eingefügt. Dabei wird das Material überstopft, das heißt er wird zum, bezogen auf dessen Volumen, überwiegenden Teil aus dem Apex Wurzelkanal hinaus in das umgebende Gewebe gedrückt. In der Folge tritt der apikale Stopp aus dem apikalen Bereich hinaus und reicht nicht mehr als 1/10 der Gesamtwurzellänge in den Wurzelkanal hinein.

Anschließend erfolgt b) ein Applizieren der Füllung. Diese umfasst den Glasfaserstift und das dentin-adhäsive Füllmaterial. Beispielsweise wird der Glasfaserstift in das Füllmaterial eingetaucht oder das Füllmaterial um diesen herum durch auftragen angebracht. Das Gemenge aus Stift und Füllmaterial wird anschließend bis zum apikalen Stopp in den Kanal eingeführt. Dabei geht das Füllmaterial mit dem Glasfaserstift eine Verbindung ein. Bei einer mehrteiligen Ausgestaltung des Stifts wird dieser durch das Füllmaterial aufgespreizt und Füllmaterial dringt in die entstandenen Zwischenräume. Dadurch entsteht zum einen eine bessere Verbindung und zum anderen werden Hohlräume vermieden. Zudem wird der Stabilisierungseffekt des Glasfaserstifts erhöht.

Schließlich wird c) das Füllmaterial ausgehärtet. Dies erfolgt bevorzugt unter zu Hilfenahme von Licht. Um eine Durchhärtung zu gewährleisten und einen Anteil nicht polymerisierter Monomere bzw. Oligomere klein zu halten kann entweder in einzelnen Schichten gehärtet werden oder aber durch die Wahl der erfindungsgemäßen Glasfaserstifte eine Beleuchtung im Innern erzielt werden. Hierbei ist allerdings erforderlich, dass beim Aushärten ein Teil des zweiten Endes des Glasfaserstifts der Härtelampe zugänglich ist. Da Licht wird anschließend zu einem Teil in den Glasfaserstift eingeleitet und von dort durch ihn hindurch bis zum ersten Ende, wo es insbesondere auch seitlich, austritt und das Komposit härtet.

Optional erfolgt zwischen Schritt a und b ein weiter Schritt, insbesondere der Schritt des Einbringens eines selbstadhäsiven Bondings, insbesondere unter Zuhilfenahme eines auf die Länge abgestimmten Hilfsmittel, insbesondere eines sogenannten Brushes.

Das erfindungsgemäße Verfahren hat insbesondere den Vorteil einer Zeitersparnis, da im Vergleich zu den üblichen Verfahren zusätzliches schmieriges Versäubern der coronalen Kavität und ein revidieren von 50 bis 70 % der neu gelegten Wurzelkanalfüllung entfallen und somit ein Kunststoffaufbau in nur einem Schritt erfolgt. Ferner dient es Schulungszwecken um das Verfahren für die Anwendung in-vivo komplikationsfrei durchführen zu können.

Die Anwendung im lebenden Bereich hat den Vorteil, dass eine bakteriendichte Wurzelfüllung erst möglich wirf, die wegen Nebenwirkungen der Komposite bislang nicht möglich ist. Der apikale Stopp und insbesondere die geringe Ausdehnung desselben im Wurzelkanal ermöglicht diese sowie eine maximale Stabilisierung der Wurzel durch das ausgehärtete Komposit im Vergleich zur Verwendung eines, abbaubaren, Knochenklebers.

Bei Verwendung der Set-Alternative in dem die Wurzelkanalfüllzusammensetzung bereits im Wesentlichen einstückig vorliegt wird diese mit dem apikalen Stopp voran in den Wurzelkanal eingeführt, derart, dass der apikale Stopp den kanal apikal verschließt und insbesondere aus dem Wurzelende in das umgebende Gewebe hinaus tritt. Das im Wurzelkanal verbleibende Ende reicht nicht mehr als 1/10 der Gesamtkanallänge in diesen hinein.

Dabei wird die Arbeitslänge mit Mitteln gemessen und eingehalten, die dem Fachmann bekannt sind. Hierzu sind beispielsweise die röntgenologische bzw. elektronische Messung zu nennen, die dann mit Hilfe einer Skalierung bzw. eines Stoppers an jedem Arbeitsmittel, insbesondere an der Einbringhilfe des apikalen Stopps bzw. dem Glasfaserstift, eingehalten wird.

Bei dem erfindungsgemäßen Verfahren werden insbesondere die Verwendung der Materialien in den oben beschriebenen Ausführungen bevorzugt. Mit anderen Worten alle voranstehenden Gegenstände und Verwendungen der Erfindung und deren Ausführungsformen sind vorteilhaft zu kombinieren. Zudem betreffen die Ausführungen zu bestimmten Gegenständen und Verwendungen der Erfindung stets auch die anderen Gegenstände und Verwendungen und deren Ausführungsformen.

Weitere bevorzugte Ausgestaltungen der Erfindung ergeben sich aus den übrigen, in den Unteransprüchen genannten Merkmalen.

Die verschiedenen in dieser Anmeldung genannten Ausführungsformen der Erfindung sind, sofern im Einzelfall nicht anders ausgeführt, mit Vorteil miteinander kombinierbar.

Die Erfindung wird nachfolgend in Ausführungsbeispielen anhand der zugehörigen Zeichnungen erläutert. Es zeigen:
- Figur 1: eine schematische Prinzipskizze einer Wurzelkanalfüllungszusammensetzung in einer bevorzugten Ausgestaltung,
- Figur 2: ein Röntgenbild einer in einen Wurzelkanal applizierten Wurzelkanalfüllungszusammensetzung in der bevorzugten Ausgestaltung und
- Figur 3: eine vergrößerte Darstellung eines Ausschnitts des Röntgenbildes aus Figur 2.

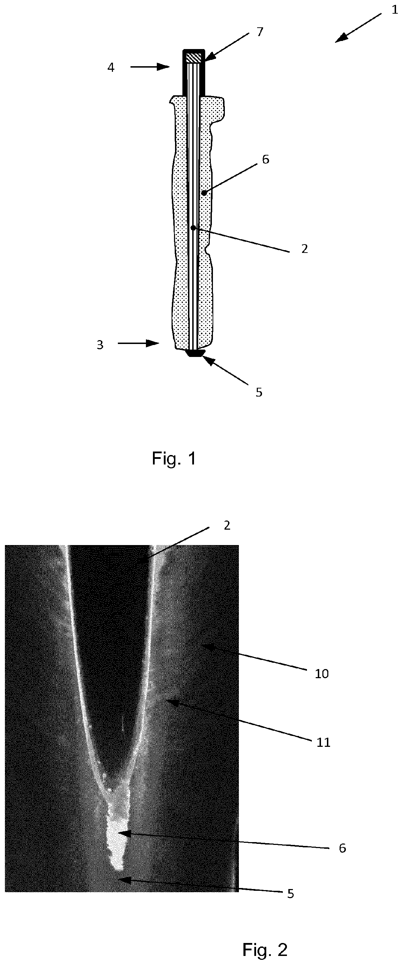

Figur 1 zeigt eine schematische Prinzip Skizze einer Wurzelkanalfüllungszusammensetzung 1 in einer bevorzugten Ausgestaltung der Erfindung. Gezeigt ist ein Glasfaserstift 2 der einteiligen oder mehrteilig ausgestaltet sein kann. Der Glasfaserstift 2 weist ein erstes Ende 3 und ein zweites Ende 4 auf. Insbesondere in mehrteilige Ausgestaltung verjüngt sich der Glasfaserstift 2 zum ersten Ende 3 hin. Am ersten Ende 3 des Glasfaserstiftes 2 ist ein apikale Stopp 5 angeordnet. Dieser ist aus einem physiologischen Material gefertigt und umfasst bevorzugt einen Knochenkleber, wie beispielsweise Hydroxylappatit und/oder ein alkyl-Cyanoakrylat. Am zweiten Ende 4 des Glasfaserstiftes 2 ist in der gezeigten Ausführungsform eine Halterung 7 angeordnet. Diese Halterung 7 ist grundsätzlich optional ermöglicht aber eine Stabilisierung insbesondere dann wenn der Glasfaserstift 2 mehrteiliger Form ausgestaltet ist. Um den Glasfaserstift 2 herum ist ein Füllungsmaterial 6 angeordnet welches aus einem dentin-adhäsiven Material besteht. Das dentin- adhäsive Material hat den Vorteil, dass es bei Applikation in einem geöffneten Zahnwurzelkanal in die Dentintubuli hinein gelangt und somit den Kanalbakterien dicht verschließt und dem geöffneten Wurzelkanal nach Schließung wieder Stabilität verleiht. Das Füllmaterial 6 kann ferner Partikel aufweisen, welche dem Effekt einer Schrumpfung des Füllmaterials 6 reduzieren und gleichzeitig die Stabilität der Füllung erhöhen.

In Figur 2 ist ein hochaufgelöstes Röntgenbild eines mit der erfindungsgemäßen Wurzelkanalfüllungszusammensetzung 1 gefüllten Wurzelkanals gezeigt. In dieser Ausgestaltung weist das Füllmaterial 6 einen röntgenopaken Zusatz auf, der es ermöglicht die Anordnung des Füllmaterials im Zahnwurzelkanal im Röntgenbild sichtbar zu machen. Gezeigt ist der Glasfaserstift 2, welcher in der gezeigten Ausführungsform zum apikalen Ende 3 hin chronisch verläuft. Dies wird insbesondere durch die mehrteilige Ausgestaltung des Glasfaserstifts 2 erreicht, so das mehrere Fasern des Glasfaserstift 2 in unterschiedlicher Länge ausgestaltet sind, sodass der Durchmesser des gesamten Glasfaserstift 2 vom apikalen Ende 3 her zum zweiten Ende 4 des Glasfaserstift 2 hin ansteigt. Deutlich wird, dass das Füllmaterial 6 auch in einem sich stark verjüngen Bereich des Wurzelkanals eindringt der den apikalen Bereich des Zahnwurzelkanals darstellt, ohne jedoch aus diesem heraus zu treten. Vielmehr ist der Ausgang des apikalen Bereichs hin zum Zahnhalteapparat (nicht gezeigt) durch den apikalen Stopp 5 verschlossen. In Figur 2 ist der apikale Stopp 5 nur als Schatten zu sehen, da diesem keine röntgenopaken Zusätze beigefügt sind. Deutlich wird jedoch, dass der apikale Stopp 5 nur zu einem sehr geringen Teil, nämlich zu nicht mehr als einem Zehntel, insbesondere zu nicht mehr als einem Zwanzigstel der Gesamtwurzelkanallänge in den Wurzelkanal hineinreicht.

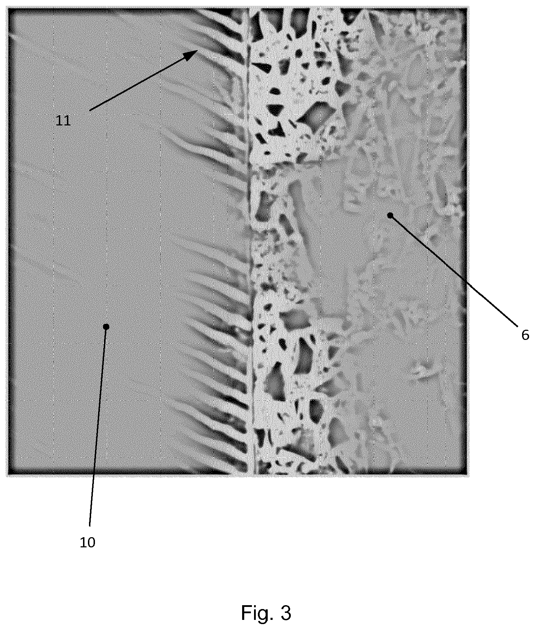

In Figur 3 ist ein Ausschnitt der in Figur 2 gezeigten Wurzelkanalfüllung gezeigt. Besonders deutlich zu sehen ist die Adhäsion des Füllmaterials in die Dentintubuli 11 des Zahnwurzelkanals, sowie die dem Füllmaterial 6 zugesetzten Füllstoffe.

Die erfindungsgemäße Wurzelkanalfüllung Zusammensetzung 1 sowie das Verfahren zu dessen Applikation ermöglicht einer bakteriendichte und stabile Wurzelkanalfüllung.

Ausführungsbeispiel zur Durchführung des erfindungsgemäßen Verfahrens in einer bevorzugten nicht beschränkenden Ausführungsform:
Als Voraussetzung liegt ein aufbereiteter und getrockneter Kanal vor. Anschließend wird der erfindungsgemäße apikale Stopp eingebracht, gefolgt von einem selbstätzenden Bonding. Nach kurzer Trocknungszeit wird der erfindungsgemäße Glasfaserstift mit dem daran angeordneten Füllmaterial, insbesondere dem Komposit in den aufbereiteten und apikal verschlossenen Kanal eingebracht. Abschließend erfolgt nach Härtung des Komposits der Aufbau des Zahns im oberen Bereich sowie ein Modelieren der Außenflächen, in einem zusammenhängenden Arbeitsschritt, also ohne längere Wartezeiten.

### Anwendungsbeispiel:

Detaillierte Anbringung der erfindungsgemäßen Wurzelkanalfüllungszusammensetzung in einer bevorzugten Ausgestaltung unter Verwendung des Sets. Dabei lagen die Einzelbestandteile getrennt voneinander verpackt vor. Das Set umfasst in diesem Beispiel zu der Grundausstattung aus mehrteiligem Glasfaserstift, 2-K-Füllmaterial mit Füllstoffen und physiologischem Stopp in Form eines Hydroxylapatit umfassenden Collagenstopfens oder einer pastös vorliegenden alkyl-Cyanoacrylatzusammensetzung ferner ein insbesondere slebstadhäsives/selbstätzendes Bonding umfassend ein Ätzmittel in Form von Phosphorsäure und einen dentin-adhäsiven Primer.

Zunächst wird ein apikaler Stopp beispielsweise aus einem Hydroxylapatit oder ein alkyl-Cyanoacrylat aufweisenden Pfropfes in den Wurzelkanal eingebracht und mit geeignetem Werkzeug derart überstopft, dass der im Kanal verbleibende Stopp nicht mehr als 1/10, insbesondere nicht mehr als 1/20 in der Gesamtkanallänge in den Wurzelkanal hineinreicht.

Anschließend wird die Dentinoberfläche mit einem selbstadhäsiven Bonding benetzt.

Das Monomer, also das dentin-adhäsive Füllmaterial wird anschließend aufgetragen und durchdringt das Kollagenfasernetzwerk bis in die Dentinkanälchen. Die entstandene Hybridschicht wird durch lichtinduzierte Polymerisation ausgehärtet. Diese Polymerschicht ist nun mikromechanisch über Tags (englisch Zapfen) in den Tubuli und im durchdrungenen Kollagenfasernetzwerk verankert.

### Bezugszeichenliste

- 1: Wurzelkanalfüllungszusammensetzung
- 2: Glasfaserstift
- 3: 1. Ende
- 4: 2. Ende
- 5: apikaler Stopp
- 6: Füllungsmaterial
- 7: Halterung
- 10: Dentin
- 11: Dentintubuli

## Patentansprüche

1. Wurzelkanalfüllzusammensetzung (1) zur Anordnung in einem Zahnwurzelkanal umfassend einen längserstreckten Glasfaserstift (2) mit einem ersten Ende (3) und einem zweiten Ende (4), einen an dem ersten Ende (3) angeordneten apikalen Stopp (5) aus einem physiologischem Material, ein den Glasfaserstift (2) zumindest bereichsweise vollflächig umschließenden Füllmaterial (6), wobei das Füllmaterial (6) ein dentin-adhäsives Füllmaterial (6) umfasst, **dadurch gekennzeichnet, dass** der apikale Stopp (5) eine Länge von nicht mehr als 1/10 der Gesamtlänge des Glasfaserstifts aufweist.

2. Wurzelkanalfüllzusammensetzung (1) nach Anspruch 1, **dadurch gekennzeichnet, dass** das Füllmaterial (6) ein Komposit, insbesondere ein dual-härtendes Komposit und/oder ein selbstadhäsives Material, umfasst oder aus einem solchen besteht.

3. Wurzelkanalfüllzusammensetzung (1) nach einem der vorhergehenden Ansprüche, **dadurch gekennzeichnet, dass** der Glasfaserstift (2) ein- oder mehrteilig ausgebildet ist, wobei bei mehrteiliger Ausbildung mehrere nicht stoffschlüssig miteinander verbundene nebeneinander angeordnete längserstreckte Teile den Glasfaserstift (2) ausbilden und insbesondere über eine Halterung (7) lösbar miteinander verbunden sind.

4. Wurzelkanalfüllzusammensetzung (1) nach einem der vorhergehenden Ansprüche, **dadurch gekennzeichnet, dass** der apikale Stopp (5) ein biologisch resorbierbares Material umfasst oder aus einem solchen besteht.

5. Wurzelkanalfüllzusammensetzung (1) nach einem der vorhergehenden Ansprüche, **dadurch gekennzeichnet, dass** der apikale Stopp (5) ein alkyl-Cyanoacrylat, insbesondere mit einer Alkylkettenlänge im Bereich von C₂ bis C₈ umfasst

6. Wurzelkanalfüllzusammensetzung (1) nach einem der vorhergehenden Ansprüche, **dadurch gekennzeichnet, dass** der apikale Stopp (5) ein Alginat, eine Hyaluronsäure, ein Collagen, und/oder einen, insbesondere Cyanoacrylate, Hydroxylapatit, Acylacrylnitril, Dialkylmethylenmanolat, α-substituierte Vinylidenealkylsulfonate, Sulfonate und/oder deren Derivate enthaltenden, Knochenkleber umfasst.

7. Wurzelkanalfüllzusammensetzung (1) nach einem der vorhergehenden Ansprüche, **dadurch gekennzeichnet, dass** der apikale Stopp (5) eine Länge von nicht mehr als 1/20 der Länge des Glasfaserstifts (2) aufweist.

8. Set zur Herstellung einer Wurzelkanalfüllzusammensetzung (1) nach einem der vorhergehenden Ansprüche enthaltend einen Glasfaserstift (2), einen apikalen Stopp (5) oder dessen Vorläufer, sowie ein dentin-adhäsives Füllmaterial (6) oder dessen Vorläufer, **dadurch gekennzeichnet, dass** der apikale Stopp (5) oder dessen Vorläufer eine Länge von nicht mehr als 1/10 der Gesamtlänge des Glasfaserstifts aufweist.

9. Set nach Anspruch 8, **dadurch gekennzeichnet, dass** das Füllmaterial (6) vorgemischt und nicht oder nur teilweise ausgehärtet vorliegt.

10. Set nach einem der Ansprüche 8 bis 9, **dadurch gekennzeichnet, dass** das Füllmaterial (6) ein Komposit, insbesondere ein dual-härtendes oder lichthärtendes und/oder ein selbstadhäsives Komposit ist.

11. Set nach einem der Ansprüche 8 bis 10, ferner aufweisend einen dentin-adhäsiven Primer.

12. Set nach einem der Ansprüche 8 bis 11, wobei der Glasfaserstift (2), der apikale Stopp (5) oder dessen Vorläufer und das dentin-adhäsive Füllmaterial (6) oder dessen Vorläufer räumlich getrennt voneinander, insbesondere einzeln verpackt, in dem Set angeordnet oder in Form der Wurzelkanalfüllzusammensetzung (1) nach einem der Ansprüche 1 bis 7 vorliegt, wobei bei letzterem das an dem Glasfaserstift (2) angeordnete Füllmaterial (6) zusätzlich derart isoliert umschlossen ist, dass es am Glasfaserstift (2) insbesondere mit Kontakt zu diesem fixiert ist, und insbesondere hermetisch und steril abgeschlossen, ist.

13. Set nach einem der Ansprüche 8 bis 12, wobei das Set jedes der Bestandteile in einer Menge umfasst, die die Füllung je eines Zahns ermöglicht.

14. Verfahren zum ex-vivo Anbringen einer Wurzelkanalfüllzusammensetzung (1) nach einem der Ansprüche 1 bis 7 in einem über den apikalen Bereich hinaus eröffneten Wurzelkanal umfassend die folgenden Schritte in der angegebenen Reihenfolge:
a) Anbringen des physiologischen apikalen Stopps im Apex, wobei der apikale Stopp durch Überstopfen optional aus dem apikalen Bereich hinaustritt und nicht mehr als 1/10 der Gesamtwurzellänge in den Wurzelkanal hineinreicht,
b) Applizieren eines Füllmaterials (6) im Wurzelkanal umfassend einen Glasfaserstift (2) und ein dentin-adhäsives Füllmaterial (6), und
c) aushärten des Füllmaterials (6).

## Claims

1. Root canal filling composition (1) for placement in a dental root canal, comprising an elongated fibreglass post (2) having a first end (3) and a second end (4), an apical stop (5) made from a physiological material arranged on the first end (3), a filling material (6) which surrounds the entire surface of at least a portion of the fibreglass post (2), wherein the filling material (6) comprises a dentin-adhesive filling material (6), **characterized in that** the apical stop (5) has a length of not more than 1/10 of the total length of the fibreglass post.

2. Root canal filling composition (1) according to Claim 1, **characterized in that** the filling material (6) comprises a composite, in particular a dual-curing composite and/or a self-adhesive material, or consists of such.

3. Root canal filling composition (1) according to any one of the preceding claims, **characterized in that** the fibreglass post (2) is constructed from one or more parts, wherein in the multipart structure the fibreglass post (2) is formed by a plurality of elongated parts which are arranged side by side and not materially bonded to each other, and in particular are connected to each other detachably via a holder (7).

4. Root canal filling composition (1) according to any one of the preceding claims, **characterized in that** the apical stop (5) comprises a bioresorbable material, or consists of such.

5. Root canal filling composition (1) according to any one of the preceding claims, **characterized in that** the apical stop (5) comprises an alkyl cyanoacrylate, in particular having an alkyl chain length in the range from C₂ to C₈.

6. Root canal filling composition (1) according to any one of the preceding claims, **characterized in that** the apical stop (5) comprises an alginate, a hyaluronic acid, a collagen, and/or a bone adhesive that contains in particular cyanoacrylates, hydroxyapatite, acyl acrylonitrile, dialkylmethylene manolate, α-substituted vinylidene alkyl sulfonates, sulfonates and/ derivatives thereof.

7. Root canal filling composition (1) according to any one of the preceding claims, **characterized in that** the apical stop (5) has a length of not more than 1/20 of the length of the fibreglass post (2).

8. Kit for the preparation of a root canal filling composition (1) according to any one of the preceding claims, including a fibreglass post (2), an apical stop (5) or precursor thereof, and a dentin-adhesive filling material (6) or precursor thereof, **characterized in that** the apical stop (5) or precursor thereof has a length of not more than 1/10 of the total length of the fibreglass post.

9. Kit according to Claim 8, **characterized in that** the filling material (6) is premixed and is in the uncured or only partly cured state.

10. Kit according to one of Claims 8 to 9, **characterized in that** the filling material (6) is a composite, in particular a dual-curing or light-curing and/or a self-adhesive composite.

11. Kit according to any one of Claims 8 to 10, further including a dentin-adhesive primer.

12. Kit according to any one of Claims 8 to 11, wherein the fibreglass post (2), the apical stop (5) or precursor thereof and the dentin-adhesive filling material (6) or precursor thereof are spatially separated from each other, in particular packaged individually, arranged in the kit or are in the form of the root canal filling composition (1) according to any one of Claims 1 to 7, wherein in the latter case the filling material (6) arranged on the fibreglass post (2) is additionally enclosed in isolating manner such that it is fixed to the fibreglass post (2), in particular with contact therewith, and in particular is sealed hermetically and to preserve a sterile state.

13. Kit according to any one of Claims 8 to 12, wherein the kit comprises each of the ingredients in a quantity that allows the filling of one tooth in each case.

14. Method for *ex-vivo* placement of a root canal filling composition (1) according to any one of Claims 1 to 7 in a root canal opened beyond the apical area, comprising the following steps in the order as indicated:
a) attaching the physiological apical stop in the apex, wherein the apical stop through overfilling optionally escapes from the apical area and does not penetrate more than 1/10 of the total root length into the root canal,
b) applying a filling material (6) in the root canal comprising a fibreglass post (2) and a dentin-adhesive filling material (6), and
c) curing the filling material (6).

## Revendications

1. Composition de comblement de canal radiculaire (1) destinée à être disposée dans un canal radiculaire dentaire comprenant une tige en fibre de verre étirée en longueur (2) dotée d'une première extrémité (3) et d'une seconde extrémité (4), une butée apicale (5) en matériau physiologique disposée à la première extrémité (3), un matériau de comblement (6) entourant du moins par endroits sur toute sa surface la tige en fibre de verre, le matériau de comblement (6) comprenant un matériau de comblement (6) adhérant à la dentine, **caractérisée en ce que** la butée apicale (5) présente une longueur ne dépassant pas 1/10 de la longueur totale de la tige en fibre de verre.

2. Composition de comblement de canal radiculaire (1) selon la revendication 1, **caractérisée en ce que** le matériau de comblement (6) comprend un composite, en particulier un composite à double durcissement et/ou un matériau auto =adhésif ou est composé de celui-ci.

3. Composition de comblement de canal radiculaire (1) selon une des revendications précédentes, **caractérisée en ce que** la tige en fibre de verre (2) est réalisée en une ou plusieurs pièces, plusieurs pièces étirées en longueur juxtaposée non connectées entre elles par correspondance de matière constituant la tige en fibre de verre (2) dans le cas d'une conformation à plusieurs pièces et étant en particulier entre elles de manière dissociable par un support (7).

4. Composition de comblement de canal radiculaire (1) selon une des revendications précédentes, **caractérisée en ce que** la butée apicale (5) comprend un matériau biologiquement résorbable ou est composée de celui-ci.

5. Composition de comblement de canal radiculaire (1) selon une des revendications précédentes, **caractérisée en ce que** la butée apicale (5) comprend un alkyle cyanoacrylate, en particulier ayant une longueur de chaîne alkyle dans la plage de C₂ à C₈.

6. Composition de comblement de canal radiculaire (1) selon une des revendications précédentes, **caractérisée en ce que** la butée apicale (5) comprend un alginate, un acide hyaluronique, un collagène, et/ou, en particulier cyanoacrylate, hydroxylapatite, acylacrylnitrile, dialkylméthylène manolate, vinylidène alkylsulfonate α- substitué, sulfonate et/ou une colle d'os contenant leurs dérivés.

7. Composition de comblement de canal radiculaire (1) selon une des revendications précédentes, **caractérisée en ce que** la butée apicale (5) présente une longueur ne représentant pas plus de 1/20 de la longueur de la tige en fibre de verre (2).

8. Ensemble de fabrication d'une composition de comblement de canal radiculaire (1) selon une des revendications précédentes, contenant une tige en fibre de verre (2), une butée apicale (5) ou ses précurseurs, de même qu'un matériau de comblement adhérant à la dentine (6) ou ses précurseurs, **caractérisé en ce que** la butée apicale (5) ou ses précurseurs présente une longueur ne dépassant pas 1/10 de la longueur totale de la tige en fibre de verre.

9. Ensemble selon la revendication 8, **caractérisé en ce que** le matériau de comblement (6) se présente sous forme pré-mélangée et non ou seulement partiellement durcie.

10. Ensemble selon une des revendications 8 à 9, **caractérisé en ce que** le matériau de comblement (6) est un composite, en particulier un composite à double durcissement ou un composite photodurcissable ou un composite autoadhésif.

11. Ensemble selon une des revendications 8 à 10, présentant en outre un primaire adhérant à la dentine.

12. Ensemble selon une des revendications 8 à 11, dans lequel la tige en fibre de verre (2), la butée apicale (5) ou son précurseur et le matériau de comblement adhérant à la dentine (6) ou son précurseur sont disposés séparés les uns des autres dans l'espace, en particulier emballés individuellement, dans l'ensemble, ou se présentent sous la forme de la composition de comblement de canal radiculaire (1) selon une des revendications 1 à 7, sachant que, pour cette dernière, le matériau de comblement (6) disposé au niveau de la tige en fibre de verre (2) est de plus entouré en étant isolé de manière à être fixé à la tige en fibre de verre (2), en particulier avec contact avec celle-ci, et en particulier fermé hermétiquement et de manière stérile.

13. Ensemble selon une des revendications 8 à 12, dans lequel l'ensemble comprend chacune des composantes en une quantité permettant à chaque fois le comblement d'une dent.

14. Procédé d'application ex vivo d'une composition de comblement de canal radiculaire (1) selon une des revendications 1 à 7 par un canal radiculaire ouvert au-delà de la zone apicale, comprenant les étapes suivantes dans l'ordre indiqué:
a) installation de la butée apicale physiologique dans l'apex, la butée apicale sortant en option par bourrage de la zone apicale et ne rentrant pas dans le canal radiculaire sur plus de 1/10 de la longueur totale de la racine,
b) application d'un matériau de comblement (6) dans le canal radiculaire, comprenant une tige en fibre de verre (2) et un matériau de comblement adhérant à la dentine (6), et
c) durcissement du matériau de comblement (6).
